(19) Europäisches Patentamt
European Patent Office
Office européen des brevets

(11) **EP 2 059 269 B1**

(12) **EUROPÄISCHE PATENTSCHRIFT**

(45) Veröffentlichungstag und Bekanntmachung des Hinweises auf die Patenterteilung:
**12.11.2014 Patentblatt 2014/46**

(21) Anmeldenummer: **07801298.6**

(22) Anmeldetag: **30.08.2007**

(51) Int Cl.:
*A61L 27/46* (2006.01)    *A61L 27/50* (2006.01)

(86) Internationale Anmeldenummer:
**PCT/DE2007/001561**

(87) Internationale Veröffentlichungsnummer:
**WO 2008/028466 (13.03.2008 Gazette 2008/11)**

(54) **PHASEN- UND SEDIMENTATIONSSTABILE, PLASTISCH VERFORMBARE ZUBEREITUNG MIT INTRINSISCHER PORENBILDUNG, BSPW. ZUM AUFFÜLLEN VON KNOCHENDEFEKTEN BZW. ZUR VERWENDUNG ALS KNOCHENERSATZMATERIAL, UND VERFAHREN ZU DEREN HERSTELLUNG**

PHASE- AND SEDIMENTATION-STABLE, PLASTICALLY DEFORMABLE PREPARATION WITH INTRINSIC PORE FORMING, INTENDED FOR EXAMPLE FOR FILLING BONE DEFECTS OR FOR USE AS BONE SUBSTITUTE MATERIAL, AND METHOD OF PRODUCING IT

PRÉPARATION PRÉSENTANT UNE STABILITÉ DE PHASES ET DE SÉDIMENTATION, DÉFORMABLE PLASTIQUEMENT, AVEC FORMATION INTRINSÈQUE DE PORES, PAR EXEMPLE POUR COMBLER DES DÉFAUTS OSSEUX OU S'UTILISANT COMME MATÉRIAU DE SUBSTITUTION OSSEUSE, ET PROCÉDÉ POUR LA PRODUIRE

(84) Benannte Vertragsstaaten:
**AT BE BG CH CY CZ DE DK EE ES FI FR GB GR HU IE IS IT LI LT LU LV MC MT NL PL PT RO SE SI SK TR**

(30) Priorität: **06.09.2006 DE 102006042142**

(43) Veröffentlichungstag der Anmeldung:
**20.05.2009 Patentblatt 2009/21**

(73) Patentinhaber: **Curasan AG**
**63801 Kleinostheim (DE)**

(72) Erfinder:
• **PETERS, Fabian**
**65933 Frankfurt Am Main (DE)**
• **HOFFMANN, Christiane**
**65933 Frankfurt Am Main (DE)**
• **HASANOVIC, Kathleen**
**65933 Frankfurt am Main (DE)**

(74) Vertreter: **Forstmeyer, Dietmar**
**BOETERS & LIECK**
**Oberanger 32**
**80331 München (DE)**

(56) Entgegenhaltungen:
**WO-A-03/028779        WO-A-03/082365**
**WO-A-2004/011053    US-A1- 2002 151 466**

Bemerkungen:
Die Akte enthält technische Angaben, die nach dem Eingang der Anmeldung eingereicht wurden und die nicht in dieser Patentschrift enthalten sind.

**Beschreibung**

Die Erfindung betrifft eine phasen- und sedimentationsstabile, plastisch verformbare Zubereitung (Implantatmaterial / Knochenersatzmaterial) mit intrinsischer Porenbildung, die bspw. zum Auffüllen von Knochendefekten und zur Augmentation verwendet werden kann, sowie ein Verfahren zu deren Herstellung.

Resorbierbare und nichtresorbierbare Implantatmaterialien auf Basis von Galciumphosphaten sind seit einiger Zeit als Knochenersatzmaterialien zum Auffüllen von Knochendefekten und zur Augmentation bekannt. Neben Formkörpern und Granulaten sind insbesondere injizierbare und knetbare Implantatmaterialien von Interesse.

Eine Methode und Komposition zur Reparatur von Knochen wird im WO 03/063686 offenbart. Bei der Komposition handelt es sich um ein Hydrogel, das aus Hyaluronsäure gebildet ist, welches mit tierischen Knochenbestandteilen, knochenähnlichem Pulver oder mit Hydroxylapatit vermischt ist. Ebenfalls kann ein wachstumsinduzierendes Peptid Bestandteil der Mischung sein.

In der Schrift EP 1 477 176 A1 wird eine formbare Knochenzusammensetzung zum Auffüllen von Knochendefekten beschrieben. Diese Zusammensetzung kann als Paste oder fließfähiges Gel ausgeführt sein und u.a. aus Knochenpulver in einem Hydrogelträger bestehen.

Die pulverförmige Komponente kann gemäß EP 1 477 176 A1 zu 25 bis 35% in dem Material enthalten sein und eine Partikelgröße von 100-850 $\mu$m aufweisen.

Der Hydrogelträger kann aus Chitosan mit einem Molekulargewicht von 100.000-300.000 Dalton bestehen und zu 1-4,5% in dem Material enthalten sein.

Aus der Schrift US 5,258,028 sind injizierbare Mikroimplantate bekannt, die aus einem Hydrogel aus Polyvinylpyrrolidon und einem plastischen Material aus Poly(dimethylsiloxan) bestehen.

Alternativ zu diesem Polymer können auch Partikel aus Calciumsalzen, wie Hydroxylapatit, biokompatible Keramiken oder biokompatible Metalle eingesetzt werden.

Die Größe der biologisch kompatiblen Partikel kann zwischen 10 und 3000 Mikrometern betragen.

Die Schrift US 4,710,76 offenbart eine Beschichtungskomposition, die aus hochporösen sphärischen Partikeln und einem resorbierbaren Binder besteht.

Die Partikel bestehen aus Tricalciumphosphat und der Binder aus Gelatine, Polyaminosäuren oder Kollagen.

Eine Komposition zur Knochenbehandlung wird in der Schrift US 4,780,450 vorgeschlagen, wobei diese polykristalliner, partikulärer Calciumphosphatkeramik, sauren, Phosphor-enthaltenden Proteinen und Type I Kollagen enthält.

Als Calciumphosphatkeramik wird in US 4,780,450 Hydroxylapatit, $\beta$-Tricalcium-phosphat und deren Mischungen aufgeführt.

Pastöses Knochenersatzmaterial wird auch in der Schrift EP 0 416 398 A1 beschrieben, wobei diese aus einer wässrigen Lösung von Pullulan, Glykol-Chitin, Carboxymethylchitin und Pectin gebildet ist, in der Calciumphosphatpartikel suspendiert sind. Als Calciumphosphate werden Hydroxylapatit, Fluorapatit, $\alpha$ - Tricalciumphosphat, ß-Tricalciumphosphat und Tetracalciumphosphat verwendet.

Knetbare Füllmaterialien werden auch in der Patentanmeldung US 2002169506 beschrieben, wobei diese aus Calciumphosphatgranulat und so genannten "kleinen Chips" bestehen.

Diese Chips werden aus Chitin gebildet und sollen nach Kontakt mit Wasser die Granula des Calciumphosphatgranulats im Sinne von Verkleben verbinden.

In der Schrift WO 03/082365 wird eine knet- und formbare Knochenersatzmasse beschrieben, die aus einem Gemisch aus calciumhaltigen Keramikpartikeln und einem Hydrogel oder einer zu einem Hydrogel quellbaren Substanz besteht, wobei die Keramikpartikel vollsynthetischen Ursprungs sind und die Mehrzahl der Keramikpartikel eine unrunde Form aufweisen.

Die einzelnen Keramikpartikel weisen eine mindestens teilweise zusammenhängende, poröse Struktur auf.

Die calciumhaltigen Keramikpartikel können aus Dicalciumphosphat-Dihydrat, Dicalciumphosphat, $\alpha$-TCP, ß-TCP, Calcium-defizientem Hydroxylapatit, Hydroxylapatit, Carbonatapatit, Chlorapatit, Whitlockit, Tetracalciumphosphat, Oxyapatit, Calciumpyrophosphat und Octacalciumphosphat bestehen.

Die zu Hydrogelen quellbaren Substanzen können Polyaminosäuren, Polysaccharide, Polylipide, Nucleotide oder Kombinationen derselben als Bausteine enthalten.

Ein injizierbares, auf Calcium basierendes, bioresorbierbares Knochenersatzmaterial offenbart die Patentanmeldung US 2003055512. Dabei handelt es sich bei dem Material um ein Gemisch aus Calciumsulfat und einem Phosphatzement-Pulver, das nach Vermischung mit Wasser aushärtet.

Eine Paste zur Behandlung von Knochen ist aus der Schrift WO 2004011053 bekannt.

Diese Paste besteht aus einem auf Hyaluronsäure basierenden Carrier-Gel und darin suspendiertem synthetischen Hydroxylapatit oder aus Knochenmaterial präpariertem Hydroxylapatit.

Die Publikation "Bioresorbable composite bone paste using Polysaccharide based nano hydroxyapatite" (R. Murugan, S. Ramakrishna, Biomaterials 2004, 25, 3829-3835) beschreibt eine Paste mit nanopartikulärem, gefällten Hydroxylapatit.

Als Binder und Matrix für die Hydroxylapatit-Partikel werden Sole aus Chitosan, einem Polysaccharid, eingesetzt.

Die Schrift FR 28 52 249 offenbart ein Knochenfüllmaterial, speziell für die Anwendung im Zusammenhang mit Oralimplantaten, bestehend aus einer Quelle von Phosphat und Calcium und ein Stimmulanz zur Anregung der Kollagenproduktion durch Osteoblasten, bspw. in Form von Hydroxyprolin-mono oder di-palmitat.

Als Calciumphosphat-Quellen sind Hydroxylapatit, Dicalciumphosphat, $\alpha$-Tricalciumphosphat, ß-Tricalciumphosphat, Tetracalciumphosphat und Octacalciumphosphat genannt, die die Kollagenbildung anregen sollen.

Die Zusammensetzung kann in verschiedenen Formen hergestellt werden, u. a. mit einer Viskosität die die Applikation in einer Spritze zulässt.

Die Viskosität der Zusammensetzung wird mittels Kohäsionspromotoren eingesetzt, welche der Gruppe der Zellulosen, Amidone, Cyclodextrine, Alginate, Dextransulfate, Polyvinylpyrrolidone oder Hyaluronsäure angehören.

Die Korngröße der Calciumphosphate ist kleiner 100 $\mu$m und kann 50, 20, 10 oder 5 $\mu$m betragen.

Die Kombination mit Platelet Rieh Plasma (PRP) und anderen Wachstumsfaktoren ist möglich.

Die Schrift WO 03/035124 beschreibt bioaktive Materialien, Verfahren zur Herstellung und Anwendung.

Offenbart wird in dieser Schrift u.a. eine bioaktive Zusammensetzung aus einer Fibroinsuspension und optional einem porenbildenden, teilchenformigen Material.

Die Fibroinsuspension kann die Form einer Okklusion, eines Gels, einer Creme oder einer Paste aufweisen.

Das Fibroin kann aus der Fasersubstanz der Seidenfäden des Seidenspinners Bombyx mori entstammen.

Das porenbildende, partikelförmige Material kann Hydroxylapatit, Tricalciumphosphat, Korelle, Chitosan oder eine Kombination dieser Materialien sein.

Weiterhin können die porenbildenden, teilchenformigen Materialien ein Komposit darstellen, bei dem der Kern aus Calciumphosphaten oder Glaskeramiken und die umgebende Schale aus einem oder mehreren biodegradierbaren Polymeren, wie Polylactid, Polyglycolid, Poly-alphahydoxysäuren, Polyamide etc. bestehen kann.

Aus der Schrift WO 03/028779 ist ein injizierbarer Knochendefektfüller, bestehend aus Calciumsalzpartikeln, einem organischen Binder mit einer Affinität zum Calciumsalz, Zellen aus der Gruppe der Stammzellen, Knochenzellen und deren Vorläufer sowie einen pharmazeutisch akzeptablen Puffer bekannt.

Als Calciumsalze kommen bei diesem Knochenfüller Calciumphosphatpartikel, wie bspw. Monetit ($CaHPO_4$), Brushit ($CaHPO_4 \cdot 2H_2O$), Calciumpyrophosphat, aber auch Calciumcarbonat und deren Kombinationen zum Einsatz. Sie können auch aus Hydroxylapatit und ß-TCP oder deren Gemischen bestehen.

Der Partikeldurchmesser der Calciumsalze liegt im Bereich von 100 bis 600 $\mu$m, vorzugsweise im Bereich von 200 bis 400 $\mu$m.

Als organische Binder sind Alginate, Dextrane, Zellulose und ihre Derivate, Plasma, biogene Binder, Hyaluronsäure und deren Kombinationen benannt.

Bevorzugt werden Hyaluronsäure, Natriumalginat, Natriumcarboxymethylcellulose, Dextran, Fibrinkleber und Transglutaminase.

In der Schrift WO 03/028779 wird dargelegt, dass die besten Effekte mit Natriumalginat erzielt werden.

Die Bindermengen liegen gemäß dieser Schrift im Bereich von 0,5 bis 10 Masse-%, vorzugsweise im Bereich von 3 bis 7 Masse-%.

Als Puffer findet ein Phosphatpuffer (PBS) Verwendung.

Der Feststoffgehalt liegt im Bereich von 30 bis 70, vorzugsweise im Bereich von 40 bis 60 Masse-%.

Pasten mit einer Viskosität von 30.000 bis 100.000 Centipoise sind gemäß WO 03/028779 gut injizierbar.

Die verwendeten Nadeln besitzen einen Durchmesser von 2 bis 5 mm und eine Länge von 5 bis 20 mm.

Die Angaben zu der für die Injektion erforderlichen Kraft werden in Abhängigkeit verschiedener Parameter dargestellt.

Neben den Zellen, die auf der Oberfläche der Calciumphosphatpartikel angesiedelt sind, können zusätzlich Wachstumsfaktoren anwesend sein.

Aus der Schrift WO 02/058755 ist ein injizierbares poröses Knochenersatzmaterial bekannt, welches in situ seine Porosität erhöhen kann.

Dieses Material besteht aus einer knochenähnlichen Verbindung und einem hydrophoben Träger.

Die knochenähnliche Verbindung kann aus Calciumphosphaten, Kaliumphosphat, Calciumsulfat, Hydroxylapatit, bioaktiven Gläsern oder Kombinationen daraus bestehen.

Die hydrophoben Substanzen können aus Proteinen, Glycoproteinen, Polyestern, Polyanhydriden, Polyaminen, wachsartigen biodegradierbaren Polymeren, wie Polyglycolid und deren Kombinationen bestehen.

Weiterhin können gemäß WO 02/058755 wässrige Komponenten sowie Gemische aus einer degradierbaren Komponente und den oben genannten knochenähnlichen Substanzen zur Anwendung kommen.

Die biodegradierbaren Komponenten können aus Polyhydroxy-polyestern, Albumin, Kollagen, Proteinen, Polysacchariden, Glycoproteinen und deren Kombinationen bestehen.

Zur Herstellung der Porosität wird eine Gasbildende Komponente, beispielsweise Wasserstoffperoxid und/oder Peroxidase vorgeschlagen.

Die Schrift WO 01/41821 beschreibt eine injizierbare, selbsthärtende Mischung, die eine Bildung ausgehärteter Biomaterialien mit breiten Variationen der Eigenschaften ermöglicht.

Diese Mischung besteht aus einer wasserbasierenden flüssigen Komponente, mindestens einem kationischen Polymer und einem Monophosphatsalz mit einem pH- Wert im Bereich von 6,5 bis 7,4. Diese flüssige Phase soll gemäß WO 01/41821 endotherme gelbildende Eigenschaften aufweisen.

Die zweite Komponente der Mischung besteht aus mindestens zwei Calciumphosphaten aus den Apatiten, Octacalciumphiosphaten, Amorphen Calciumphosphaten, Tetracalciumphosphaten, Tricalciumphosphaten, Dicalciumphosphaten und Monocalciumphosphaten.

Das kationische Polymer ist zu 0,1-5,0 Gew.-% in der ersten Komponente enthalten und kann aus Polysacchariden, Polypeptiden oder synthetischen Polymeren, Chitin oder Chitosan bestehen.

Das Monophosphatsalz kann aus Natrium-Kalium-, Magnesium-, Mangan- oder Eisenphosphaten verschiedenster Stöchiometrie zusammengesetzt sein.

Als wasserlösliches Polymer sind verschiedene modifizierte Zellulosen, Polyethylenglycol, Polyvinylalkohol, organische Polyole, Glycol-Oligomere, Zucker oder Glycerin erwähnt. Die Mischung kann ferner Wachstumsfaktoren beinhalten.

Die Schrift EP 1 475 109 A1 offenbart eine Zusammensetzung zur injizierbaren Applikation osteogener Proteine bestehend aus einer pharmazeutisch akzeptablen Mischung aus dem osteogenen Protein und einer hämostatischen Gelatine-Schaum-Paste.

Diese Zusammensetzung kann außerdem Tricalciumphosphat beinhalten. Die osteogenen Proteine können aus der BMP-Familie, vorzugsweise BMP-2 und OP-I entstammen.

Die TCP-Partikel sind mikroporös und weisen eine Partikelgröße von 45-125 $\mu$m auf, die durch eine 18-gauge Nadel (entspricht 1,2 mm Durchmesser und 40 mm Länge) injiziert werden kann.

Weiterhin werden in dieser Schrift als Agens zur kontrollierten Freisetzung der Proteine auch Alginate oder Zellulosen offenbart.

Aus der Schrift WO 01/41824 ist ein mit Magnesiumsalzen stabilisierter hydraulischer Brushitzement bekannt. Der Brushitzement ist aus einem basischen Calciumphosphat, einer zweiten Komponente, bestehend aus einem sauren Calciumphosphat, einer dritten Komponente, bestehend aus Wasser, und eine vierte Komponente zur Kontrolle der Verfestigungsreaktion, bestehend aus einem Magnesiumsalz herstellbar.

Die basischen Calciumphosphate können gemäß der Schrift WO 01/41824 aus der Klasse der Tricalciumphosphate und Apatite stammen.

Die saure Phosphatkomponente kann aus Monocalciumphosphaten bestehen. Die vierte Komponente, von 0,001 bis 60 Gew.-% im Material enthalten, kann aus diversen Magnesiumphosphaten und Magnesiumsalzen organischer Verbindungen stammen.

Die flüssige Komponente kann ebenfalls Schwefelsäure oder Phosphorsäure enthalten.

Zur Kontrolle der Fließeigenschaften der Zemente können gemäß der Schrift WO 01/41824 Additive zugesetzt werden. Diese können aus Polysacchariden, vorzugsweise Hyaluronsäure und ihren Salzen, Dextran, Alginate, Hydroxypropylmethylcellulose, Chitosan oder Xanthan bestehen.

Der Zement kann Granulate im Durchmesserbereich von 100 $\mu$m bis 500 $\mu$m, vorzugsweise im Bereich von 200 bis 350 $\mu$m. enthalten. Diese können aus Calciumphosphat oder Gips bestehen.

Die ausgehärtete Zementmischung kann ein Ca/P-Verhältnis von 1,00 bis 1,67 aufweisen.

In der Schrift WO 00/07639 werden Knochenvorläufermischungen und Methoden zur Herstellung derselben offenbart.

Diese können injizierbare Calciumzemente aus Monocalciumphosphat-Monohydrat und ß-TCP und auch Calciumpyrophosphat und Calciumsulfat enthalten.

Diese Calcium-Zemente können granuläre Formen im Bereich von 1 bis 500 $\mu$m aufweisen.

Zur Viskositätssteuerang können Kollagene, Methylcellulose, Biopolymere oder andere pharmazeutisch akzeptable Substanzen enthalten sein.

Zur Neutralisation des pH-Wertes der Knochenvorläufersubstanz können zusätzlich CAPS, Triethanolamin, TES, Tricin, HEPES, Glycin, PBS, Bis-Tris-Propan, $TAPS_5$ AMP und TRIS eingesetzt werden.

Die Schrift WO 00/45867 offenbart einen hydraulischen Zement mit einer Calciumquelle, Wasser und einer hydrophoben Flüssigkeit.

Diese Mixtur ist aus einer Calciumquelle und Wasser zusammengesetzt, welches, miteinander vermischt, eine selbsthärtende Zementpaste ergibt. Eine dritte Komponente besteht aus einer hydrophoben Flüssigkeit, die, in situ ausgewaschen, einen Zement mit einer offenen Makroporosität ergibt, der ein schnelles Knocheneinwachsen erlaubt.

Die hydrophobe Flüssigkeit kann aus der Gruppe der Fette oder Öle stammen. Die Calcium-Quelle kann aus der Klasse der Calciumphosphate stammen.

Das Ca/P-Verhältnis der Calcium-Quelle kann zwischen 1,0 und 1,67 liegen. Die Schrift nennt hierzu u.a. Monocalciumphosphat-Monohydrat und wasserfreies Monocalciumphosphat, Dicalciumphosphat, Octacalciumphosphat, Alpha- und Beta-Tricalciumphosphat, Tetracalciumphosphat, Hydroxylapatit.

Als Additiv zur Kontrolle der Fließeigenschaften der Zemente können Polymeradditive eingesetzt werden.

Diese Additive können aus der Gruppe der Polysaccharide stammen und beispielsweise modifizierte Cellulosen wie Hydroxypropylmethylcellulose, Hydroxyethylcellulose und Hyaluronsäure umfassen. Weiterhin kann noch ein Emulsionshilfsstoff aus der Gruppe der Tenside als Stabilisator eingesetzt werden. Es können pharmazeutisch und physiologisch aktive Substanzen beigemischt werden.

Aus der Schrift WO 95/21634 ist eine Biomaterialzusammensetzung und ein Verfahren zu ihrer Herstellung bekannt.

Die Zusammensetzung kann 40 bis 75 Gew.-% ß-Tricalciumphosphat und Hydroxylapatit im Verhältnis von 20 zu 80 bis 70 zu 30 enthalten, sowie Calciumtitanphosphat ($CaTi_4(PO_4)_6$) und 60 bis 25 Gew.-% einer flüssigen Phase, bestehend aus einer wässrigen Lösung eines Cellulosederivates.

Die Schrift erwähnt darüber hinaus auch Hydroxypropylmethylcellulose. Die Größe der Granulate liegt bei 80 bis 200 $\mu$m. Das Material ist steril, bereit zur Benutzung und injizierbar.

US 5,352,715 offenbart eine injizierbare Implantatzusammensetzung, umfassend Kollagen und biokompatible Keramikpartikel in einem pharmazeutisch akzeptablem flüssigem Träger, wobei die Keramikpartikel nach ihrer Größe so ausgewählt sind, dass sie eine Größenverteilung im Bereich von 50 $\mu$m und 250 $\mu$m aufweisen.

Der Nachteil vieler dieser bekannten Gemische ist, dass diese nicht über einen längeren Zeitraum homogen bzw. stabil vorliegen.

Hinzu kommt, dass einige Gemische aufgrund ihrer Beschaffenheit nicht durch Kanülen applizierbar sind.

Bei den Calcmmphosphat-haltigen Zusammensetzungen gemäß dem Stand der Technik besteht das Problem, dass sich über einen längeren Zeitraum eine relativ feste, nicht mehr knetbare Calciumphosphat-Phase unter einer wässrigen Phase absetzt, was besonders bei der gewerblichen Anwendbarkeit vorgefertigter Mischungen nachteilig ist.

Durch die vorliegende Erfindung sollen die aufgezeigten Mängel des Standes der Technik auf einfache Art und Weise behoben und ein phasen- und sedimentationsstabiles, plastisch verformbares Implantatmaterial mit intrinsischer Porenbildung bereit gestellt werden, das in zähflüssiger bis pastöser Konsistenz vorliegt, das aufwandgering realisiert werden kann und durch Injektion in einen Knochendefekt eingebracht werden kann.

Weiterhin ist es Aufgabe der Erfindung, ein Verfahren zur Herstellung dieses Materials anzugeben.

Die vorstehend genannte Aufgabe wird durch die Merkmale des ersten und achtzehnten Patentanspruchs gelöst. Vorteilhafte Ausgestaltungen sind den untergeordneten Ansprüchen entnehmbar.

Das Wesen der Erfindung besteht darin, dass Partikel mit einem Durchmesser in einem Größenbereich, der größer als ein phagocytierbarer Partikel (Die Publikation F. Peters, D. Reif, "Functional Materials for Bone Regeneration from Beta-Tricalcium Phosphate", Mat.-wiss. u. Werkstofftech. 2004, 35 (4), 203-207 beschreibt unter Kapitel 2 "particle size and mechanical properties" über Biomaterialien aus Beta-Tricalciumphosphat, dass die durchschnittliche Partikelgröße im Bereich von 7-10 $\mu$m liegen sollte, um die Phänomene der Phagocytose zu vermeiden) und der auf den jeweiligen Durchtrittsdurchmesser von gängigen Injektionskanülen angepasst ist, verwendet und mit einer Bindersubstanzen unter gleichzeitigem Zusatz stabilisierender, die Sedimentation unterdrückender Stoffe, innig vermischt werden.

Die Bindersubstanzen sind dabei organischer Natur und sind aus der Gruppe der Algiate, Stärken, Polysaccharide, Cellulosen, modifizierten Cellulosen, Hyaluronsäuren und deren Salze, Gelatine, Kollagene, Polyacrylsäuern, wässrigen oder alkoholischen Lösungen hiervon, Dextrane, Polyethylenglycole und deren Gemische ausgewählt.

Unter Verwendung von wasserfreien Gemischen von flüssigen Polyethylenglykolen und Calciumphosphatpartikeln lassen sich gemäß der vorliegenden Erfindung stabile, fließfähige Suspensionen herstellen. Ebenso sind stabile Suspensionen mit wässrigen Lösungen von den oben genannten Polysacchariden, Alginaten, Stärken, modifizierten Cellulosen, Proteinen und Proteingemischen gemäß der Erfindung herstellbar.

Durch den erfindungsgemäßen Zusatz von Kohäsionspromotoren, besonders vorteilhaft Hydrogelen, zu der Zusammensetzung wird ein Abgleiten der Keramikpartikel gegeneinander bewirkt. Gleichzeitig verhindern die erfindungsgemäß zugesetzten Kohäsionspromotoren das Absetzen (die Sedimentation) einzelner Komponenten in der Zusammensetzung, so dass diese über längere Zeit als homogene Suspension aufrechterhalten wird.

Die leicht lösliche Hydrogel-Komponente dient im Defekt als intrinsischer Porenbildner. Die Zwischenräume zwischen den Partikeln, deren Größe abhängig von der Partikelgröße und Partikelgrößenverteilung ist, dienen dem Einwachsen von Adern und Gewebe. Die leichter lösliche Hydrogel-Matrix weitet das Partikelhaufwerk zusätzlich auf und dient gleichzeitig als Kohäsionspromotor um die Injizierbarkeit zu gewährleisten. Die Hydrogel-Matrix befindet sich zwischen den Partikeln. Beim Einbringen in den Defekt koaguliert Blut Calcium-indiziert an der Oberfläche des Partikelhaufwerks. Die leichter lösliche Hydrogel-Komponente wird innerhalb eines kurzen Zeitraums resorbiert, das durch fibröses Gewebe stabilisierte Partikelhaufwerk bleibt zurück. Dieses Haufwerk aus resorbierbarer Biokeramik mit großen interpartikulären Zwischenräumen erfüllt nunmehr alle Anforderungen an ein Knochenersatzmaterial bzgl. Porosität, Phasenreinheit und Resorbierbarkeit.

Unter dem Begriff gering löslich wird folgendes verstanden. Die gering lösliche Substanz löst sich bei Raumtemperatur in Wasser in einer maximalen Konzentration von 100 mg/l.

Der erfindungsgemäße Zusatz von phasenreinen Stoffen, insbesondere von phasenreinem Beta-Tricalciumphosphat, zu der Zusammensetzung bewirkt eine größere Gefügestabilität, da keine Materialien mit unterschiedlichen thermischen Ausdehnungskoeffizienten oder unterschiedlicher Löslichkeit einen verfrühten partikulären Zerfall bewirken.

Die erfindungsgemäße Zusammensetzung weist Partikel mit interkonnektierenden Poren (Mikroporen < 0,01 - 50 μm) auf, so dass in vzvo eine Zellversorgung auch innerhalb der Zusammensetzung / des Biomaterials erfolgen kann. Die Flüssigkeiten (bspw. Blut oder Körperflüssigkeit) lösen außerdem ein resorbierbares/degradierbares Biomaterial (wie beispielsweise Beta-Tricalciumphosphat) von innen nach außen auf, so dass eine zeitnahe Resorption mit dem Knochenwachstum einhergehen kann. Die Kapillarkräfte, bedingt durch die interkonnektierende Mikroporosität des Biomaterials, bewirken ausserdem einen tamponierenden Effekt bei der Defektversorgung. Makroporen (>50 μm) dienen der Vaskularisierung und der Leitschiene zum Einsprossen von neu gebildeten Knochen. Porendurchmesser im Bereich von 50-100 μm zeigten hierbei gute Resultate. Lange und gewundene Poren können nicht komplett mit Knochen gefüllt werden, wenn kein Kontakt mit Versorgungsmedien stattfindet. Daher ist es notwendig, die großen Poren durch kleine Poren interkonnektierend zu gestalten. Bei großen Biomaterial-Blöcken wird diese Porosität durch Einbringen von Ausbrennstoffen, Schäumen oder Bohren erreicht. Bei Granulaten erfüllten die Funktion der Makroporen die intergranulären Zwischenräume.

Weiterhin ist durch das Einbringen von Poren bzw. durch kleine Partikel eine geringere Materialmenge pro Defektvolumen bei gleichzeitiger Funktion als Platzhalter im Knochendefekt gegeben. Durch die geringere Materialmenge und der hohen Oberfläche bei kleineren Partikeln resultiert eine erhöhte Resorptionsgeschwindigkeit.

Die Gefügestabilität ist durch die erfinderische Lösung derart optimiert, dass während der Resorption kein verfrühter Zerfall in phagocytierbare Subpartikel erfolgt.

Es ist erfindungsgemäß, dass poröse Partikel im Größenbereich von 0.1-150 μm, mit Porendurchmessern von 0,01-50 μm, aus β-Tricalciumphosphat, α-Tricalciumphosphat, Whitlockit, Tetracalciumphosphat, Octacalciumphosphat, Hydroxylapatit, Carbonatapatit vom Typ A, Carbonatapatit vom Typ B$_5$ Calcium-defizientem Hydroxylapatit, amorphem Calciumphosphat und/oder resorbierbaren Glaskeramiken bestehen können. Die Partikel weisen eine polygonal gebrochene, durch Abrieb und thermische Sinterverfahren abgerundete Form auf.

Das plastisch verformbare Implantatmaterial mit derartig ausgestalteten Partikeln liegt in einer pastösen Form vor. Die pastöse Form des Implantatmaterials ist derart gestaltet, dass sie mittels einer Injektionsspritze mit einer geraden oder auch abgewinkelten Kanüle minimalinvasiv in den Knochendefekt appliziert werden kann.
Die Durchmesser der Partikel sind auf den Kanülendurchmesser einer Injektionskanüle optimiert. Die Optimierung der Partikeldurchmesser ist so gewählt, dass das pastöse Material auch durch eine bis zu 60° abgewinkelte Kanüle applizierbar ist, gemessen an der Längsachse der Injektionsspritze.

Die erfindungsgemäßen Partikel sind in Wasser schwer löslich, biologisch aktiv und weisen eine polygongebrochene abgerundete Form auf. Durch diese Form wird ein optimaler Zusammenhalt des Partikelhaufwerks erreicht.

Wesentlich für die Erfindung ist, dass es bevorzugt aus einem Gemisch aus 60-98 Masseprozent Calciumphosphat-Partikeln und 1-30 Masseprozent einer wässrigen oder alkoholischen Lösung von Dextran und/oder Carboxymethyldextran und/oder Hyaluronsäure und/oder Dermatansulfat, Carboxymethylcellulose und/oder oxidierter Cellulose und/oder Gelatine und/oder Mischungen aus diesen aufgebaut ist. Im Sinne der Erfindung ist auch die Verwendung von Polysaacharidderivaten, wir zum Beispiel Carboxymethyldextran,

Carboxymethylhyaluronsäure und sulfatierte Hyaluronsäure. Der besondere Vorteil der Verwendung von hydrophob modifizierten Calciumphosphatpartikel liegt darin, das insbesondere anionische Polysaccharide und Polysaccharidderivate, wie Hyaluronsäure oder Carboxymethyldextran, über ihre Carboxylgruppen mit den Calciumphosphatoberflächen nur eingeschränkt wechselwirken können. Dadurch werden die rheologischen Eigenschaften positiv beeinflusst. Ebenfalls erfindungsgemäß ist, dass es bevorzugt aus einem Gemisch aus 80-98 Masseprozent Calciumphosphat-Partikeln, 1-20 Masseprozent wasserfreiem Polyethylenglykol 400 und 1-20 Masseprozent wasserfreiem Polyethylenglykol 600 aufgebaut ist. Polyethylenglykol 400 und Polyethylenglykol 600 können zusätzlich noch Oxidationsstabilisatoren enthalten.

Die plastisch verformbare Zubereitung zum Knochenaufbau / -ersatz auf Basis von Keramik-Suspensionen liegt erfindungsgemäß in einer pastösen Form vor, so dass sie durch eine Injektionskanüle applizierbar ist.

Die Partikelform und die Partikelgröße der partikulären, in Wasser gering löslichen Komponente sind dabei dem Durchmesser der Injektionskanüle angepasst.

Die mittleren Partikeldurchmesser der biologisch aktiven Substanz ist dabei erfindungsgemäß derart optimiert, dass das Gemisch durch eine abgewinkelte Kanüle applizierbar ist, wobei der Winkel bis zu 60°, vorzugsweise 45° gemessen an der Längsachse der Injektionsspritze, beträgt.

Ist die Partikelgröße der zu injizierenden Keramikpartikel nach oben hin begrenzt (Verkantung der Partikel vor und in der Kanüle), so erfährt die Partikelgröße auch zu kleinen Durchmessern eine Limitation: aus diversen Arbeiten ist bekannt, dass große Mengen von Partikeln im Bereich von unter 5 μm Durchmesser zu einer überschießenden Fremdkörperreaktion führen können. Die Partikel werden dabei von Makrophagen (Fremdkörperriesenfresszellen) aufgenommen und abtransportiert bzw. verstoffwechselt (Phagocytose). Unter anderem ist dies in folgenden Veröffentlichungen angesprochen: S. Shimizu in Biomed. Res. 1988, 9 (2), 95 und J. van der Meulen und H. K. Koerten in J. Biomed. Mater. Res. 1994, 28, 1455. Die Folge können aseptische, überschießende Fremdkörperreaktionen sein, die eine Entzündung des umgebenden Weichgewebes auslösen können. Die pH-Wert-Senkung in der Umgebung der phagocytierenden Makrophagen (Entzündungsherd) fuhrt zum Zerfall weiterer Keramik in kleine Subpartikel, die ebenfalls phagocytiert werden. In die entstehenden Lücken kann Weichgewebe einsprossen und somit eine erfolgreiche Defektheilung unterbinden.

Die erfindungswesentlichen mittleren Partikeldurchmesser weisen daher in ihrer Mehrheit einen Partikeldurchmesser oberhalb der phagocytierbaren Größe auf.

Die erfindungswesentlichen mittleren Partikeldurchmesser $d_{10}$, $d_{50}$ und $d_{90}$ der biologisch aktiven Substanz / Partikel verhalten sich zum Kanülendurchmesser k folgendermaßen:

$$k > 3/2O*d_{10}+l/2, \quad k>2/25*d_{50} \quad und \quad k>2/25*d_{90}+4/5.$$

Neben der Partikelgröße weist auch die Partikelform eine erfindungswesentliche charakteristische Eigenschaft auf. Zum Erreichen einer polygonal-gebrochenen Form werden größere Einheiten der keramischen Pulver verpresst, gesintert (gebrannt) und gebrochen. Die gebrochenen Partikel werden einem thermischen Sinterschritt unterworfen, so dass sie eine abgerundete Sinterstruktur aufweisen. So sind auf den Partikeloberflächen keinerlei scharfkantigen Bereiche mehr erkennbar, die nach Implantation zu einer Irritation des umgebenden Weichgewebes führen kann.

In einer pastösen Form ist die erfindungsgemäße Zubereitung / Mischung durch eine Injektionskanüle applizierbar, wobei die Mischung von 60-80 Gew.-%, vorzugsweise 67-75 Gew.-% β-Tricalciumphosphat, 17-37 Gew.-%, vorzugsweise 25-35 Gew.-% Wasser und 0,3-3 Gew.-%, vorzugsweise 0,5-1,5 Gew.-% Methylcellulose niedriger Viskosität aufweist.

Eine andere pastöse, durch eine Injektionskanüle applizierbare Form der erfindungsgemäße Zubereitung kann aus einer Mischung von 60-80 Gew.-%, vorzugsweise 68-76 Gew.-% ß-Tricalciumphosphat, 15-35 Gew.-%, vorzugsweise 25-31 Gew.-% Wasser und 0,1-3 Gew.-%, vorzugsweise 0,2-0,9 Gew.-% Methylcellulose niedriger Viskosität und 0,01-2 Gew.-%, vorzugsweise 0,1-0,6 Gew.-% Natriumhyaluronat oder Hyaluronsäure bestehen.

Besonders vorteilhaft ist eine Zubereitung in Form eines Gemischs aus 20-98 Masseprozent Calciumphosphat-Partikeln, 1-20 Masseprozent wasserfreiem Polyethylenglykol 400 und 1-20 Masseprozent wasserfreiem Polyethylenglykol 600.
Weiterhin sind die Kombinationen HS + Methocel + Dextran; HS + Methocel + PEG bzw. HS + Methocel + PEG + Dextran besonders vorteilhaft.

Die Dichten der erfindungsgemäße Zubereitung liegt zwischen 1,3 g/cm$^3$ und 2,1 g/cm$^3$, vorzugsweise zwischen 1,6 g/cm$^3$ und 1,9 g/cm$^3$, wobei die wasserlösliche organische Bindersubstanz mit der partikulären biologisch aktiven Substanz ein im applizierten Zustand formstabiles, poröses Haufwerk bildet, die leicht lösliche Komponente in der Defektumgebung durch fibröses Gewebe ausgetauscht wird und dadurch die poröse Partikelpackung formstabil verbleibt und langsam integriert, vaskularisiert und resorbiert wird.

Besonders vorteilhaft ist, wenn die wasserlösliche organische Bindersubstanz derart ausgestattet ist, dass sie das Absetzen der partikulären, biologisch aktiven Substanz in zwei Phasen, nämlich eine dichte, hauptsächlich aus der partikulären Substanz bestehenden und eine wässrige Phase verhindert.

Besonders vorteilhaft ist darüber hinaus, wenn die in Wasser oder alkoholischen Lösungsmitteln löslichen Hydrogel-bildenden Komponenten aus Substanzen aus der Gruppe der Alginate, Stärken, Polysaccharide, Cellulosen, modifizierten Cellulosen, Hyaluronsäuren und deren Salze, Gelatine, Kollagene, Schellack, Polyacrylsäuren, wässrigen oder alkoholischen Lösungen hiervon, Dextrane, Polyethylenglycole sowie deren Gemische stammen und die biologisch aktive Substanz aus Calciumphosphaten und/oder Natrium- und/oder Kalium- und/oder Calcium- und/oder Silicat-haltigen sauren und/oder neutralen und/oder alkalischen Biogläsern oder Gemischen aus den zuvor genannten bestehen.

Die biologisch aktive Substanz besteht auch besonders vorteilhaft aus Monocalciumphosphat-Monohydrat, wasserfreiem Monocalciumphosphat, Dicalciumphosphat-dihydrat, wasserfreiem Dicalciumphosphat, ß-Tricalciumphosphat, α-Tricalciumphosphat, Tetracalciumphosphat, Whitlockit, Octacalciumphosphat, Hydroxylapatit, Oxyapatit, Carbonatapatit vom Typ A, Carbonatapatit vom Typ B, Calcium-defizientem Hydroxylapatit, amorphem Calciumphosphat, amorphen carbonathaltigen Calciumphosphat oder Gemischen aus den zuvor genannten.

Der Vorteil der erfindungsgemäßen pastösen, injizierbaren Zusammensetzungen ist, dass die in Wasser oder alkoholischen Losungsmitteln löslichen Hydrogel-bildenden Komponenten eine kristallisationsinhibierende Eigenschaft ausweisen, so dass eine Umkristallisation des Calciumphosphat-Systems oder der anderen möglichen keramischen Komponenten in andere Phasen unter Wassereinfluss durch diese stabilisierende Komponente unterdrückt wird.

Die Zubereitung kann darüber hinaus wachstumsinduzierende Proteine enthalten und so die Knochen- oder Geweberegeneration nachhaltig fordern. Diese Wachstumsfaktoren können aus der Familie der Growth Factors (GF) oder der Bone Morphogenetic Proteins stammen. Verwendung finden dabei die Wachstumsfaktoten BMP-I bis BMP-12, besonders vorteilhaft BMP-2 und BMP-7, und/oder FGF, TGF-ß, PDGF, VEGF, IGF, HGF, PTH und / oder Gemische aus diesen.

Weiterhin können auch direkt aus patienteneigenem Blut gewonnene Wachstumsfaktor-Mischungen, wie bspw. Platelet Rieh Plasma (PRP) in der Zubereitung enthalten sein.

Die Erfindung wird nachstehend an Hand der Figuren und der Ausführungsbeispiele näher erläutert. Es zeigen:

Fig. 1: eine Ausführungsform der erfindungsgemäßen Zubereitung in Form eines in einer Hydrogel-Matrix eingelagerten Partikels;

Fig. 2: die Ausführungsform gemäß Fig. 1 mit koaguliertem Blut an der Oberfläche und durch die ausgelöste Hydrogel-Matrix entstandene Poren;

Fig. 3: eine graphische Darstellung der Messergebnisse (Röntgenpulverdiffraktogramme) hinsichtlich der Konversion- bzw. Umkristallisationsstabilität der Ausführungsform der erfmdungsgemäßen Zubereitung gemäß Fig. 1 in verschiedenen Lagerungsmedien;

Fig. 4: eine graphische Darstellung der Messergebnisse hinsichtlich der Phasenstabilität der Ausführungsform der erfindungsgemäßen Zubereitung gemäß Fig. 1;

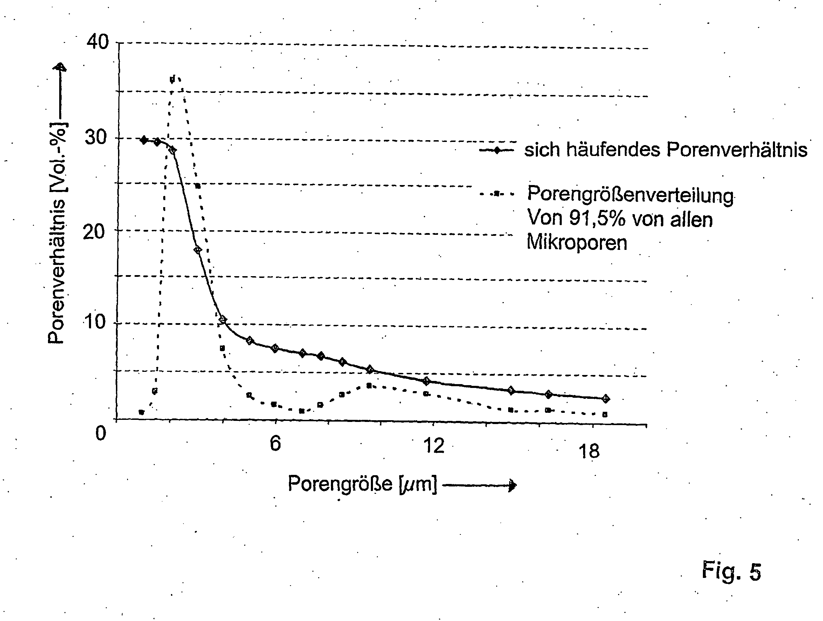

Fig. 5: eine graphische Darstellung einer Quecksilberdruckporosimetrie-Messung zum Beleg der Porenverteilung in der partikulären, schwer löslichen Komponente der Ausführungsform der erfindungsgemäßen Zubereitung gemäß Fig. 1.

Die leichter lösliche Hydrogel-Komponente dient im Knochendefekt als intrinsischer Porenbildner. Die Zwischenräume zwischen den Partikeln der Zubereitung, deren Größe abhängig von der Partikelgröße und Partikelgrößenverteilung ist, dienen dem Einwachsen von Adern und Gewebe.

Die leichter lösliche Hydrogel-Matrix weitet das Partikelhaufwerk zusätzlich auf und dient gleichzeitig als Kohäsionspromotor, um die Injizierbarkeit zu gewährleisten.

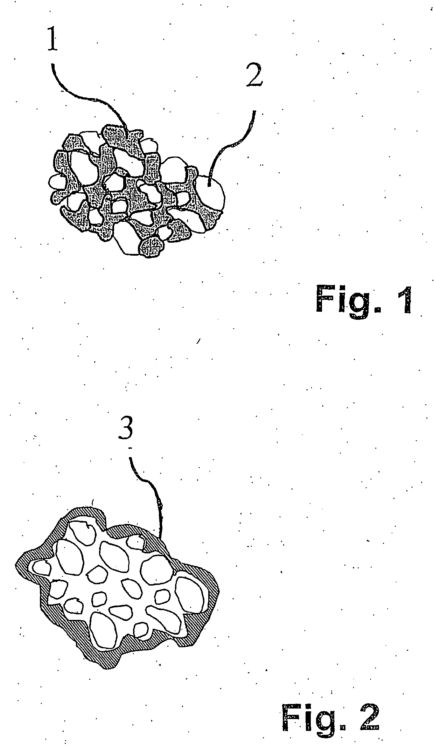

Die Hydrogel-Matrix (1) befindet sich, wie in Fig. 1 dargestellt, zwischen den Partikeln (2).

Beim Einbringen der erfindungsgemäßen Zubereitung in den Defekt koaguliert Blut (3) Calcium-indiziert an der Oberfläche des Partikelhaufwerks, was schematisch in Fig. 2 dargestellt ist.

Die leichter lösliche Hydrogel-Komponente wird innerhalb eines kurzen Zeitraums resorbiert, so dass das durch fibröses Gewebe stabilisierte Partikelhaufwerk zurückbleibt. Dieses Haufwerk aus resorbierbarer Biokeramik mit großen interpartikulären Zwischenräumen erfüllt nunmehr alle Anforderungen an ein Knochenersatzmaterial bzgl. Porosität, Phasenreinheit und Resorbierbarkeit.

Unter dem Begriff gering löslich wird folgendes verstanden: Die gering lösliche Substanz löst sich bei Raumtemperatur in Wasser in einer maximalen Konzentration von 100 mg/l.

Erfindungsgemäß ist, dass die Partikel der Zubereitung eine Größe im Bereich von 0,1 bis 150 $\mu$m aufweisen, Poren mit einem Durchmesser von 0,01-50 $\mu$m besitzen und aus ß-Tricalciumphosphat, $\alpha$-Tricalciumphosphat, Whitlockit, Octacalciumphosphat, Hydroxylapatit, Carbonatapatit vom Typ A, Carbonatapatit vom Typ B, Calcium-defizientem Hydroxylapatit, amorphem Calciumphosphat und/oder resorbierbaren Glaskeramiken bestehen können.

Die Calciumphosphatpartikel weisen eine polygonal gebrochene, durch Abrieb und thermische Sinterverfahren abgerundete Form auf.

Die o.g. Partikel im Größenbereich von 0,1 bis 150 $\mu$m, weisen Porendurchmesser von 0,01 bis 50 $\mu$m auf.

Die erfindungsgemäße, plastisch verformbare Zubereitung liegt in einer pastösen Form vor.

Die pastöse Form der Zubereitung ist derart gestaltet, dass sie mittels einer Injektionsspritze mit einer geraden oder auch abgewinkelten Kanüle minimalinvasiv in einen Knochendefekt applizierbar ist.

Die Durchmesser der Partikel sind auf den Kanülendurchmesser einer Injektionskanüle optimiert. Die Optimierung der Partikeldurchmesser ist so gewählt, dass das pastöse Material auch durch eine bis zu 60° abgewinkelte Kanüle applizierbar ist, gemessen an der Längsachse der Injektionsspritze.

Wesentlich für die Erfindung ist, dass es bevorzugt aus einem Gemisch aus 60-98 Masseprozent Calciumphosphat-Partikeln und 1-30

Masseprozent einer wässrigen oder alkoholischen Lösung von Dextran und/oder Carboxymethyldextran und/oder Hyaluronsäure und/oder Dermatansulfat, Carboxymethylcellulose und/oder oxidierter Cellulose und/oder Gelatine und/oder Mischungen aus diesen aufgebaut ist. Im Sinne der Erfindung ist auch die Verwendung von Polysaacharidderivaten, wie bspw. Carboxymethyldextran, Carboxymethylhyaluronsäure und sulfatierte Hyaluronsäure.

Ebenfalls erfmdungsgemäß ist, dass es bevorzugt aus einem Gemisch aus 80-98 Masseprozent Calciumphosphat-Partikeln, 1-20 Masseprozent wasserfreiem Polyethylenglykol 400 und 1-20 Masseprozent wasserfreiem Polyethylenglykol 600 aufgebaut ist. Polyethylenglykol 400 und Polyethylenglykol 600 können zusätzlich noch Oxidationsstabilisatoren enthalten.

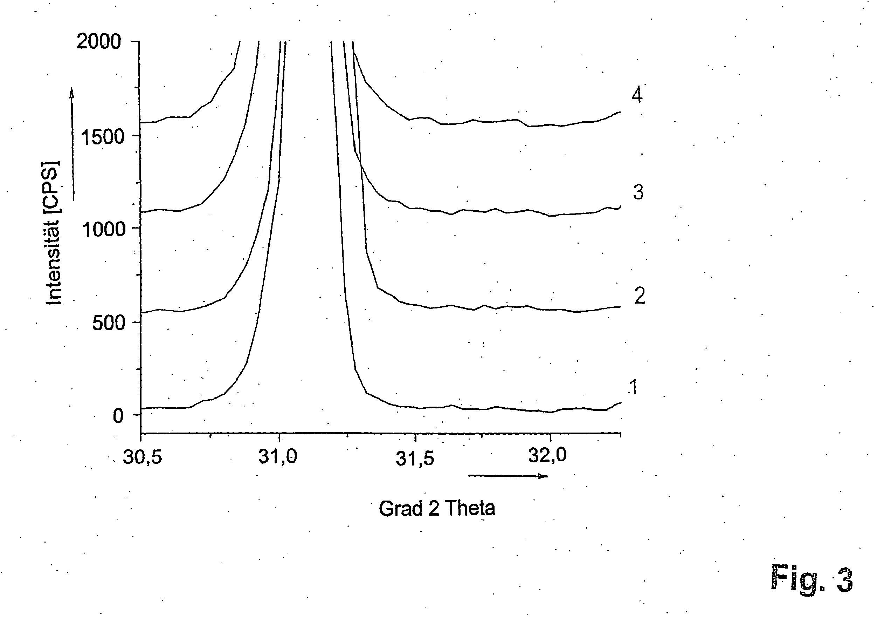

Figur 3 zeigt Detailaufnahmen der Röntgenpulverdiffraktogramme von

(1) der Ausgangscharge einer ß-TCP-Keramik

(2) ß-TCP in Hyaloronsäurelösung

(3) ß-TCPin Dextranlösung

(4) ß-TCPin Methylcellulose-Lösung nach 12 Wochen Lagerung in Wasser

Wie dieser Dokumentation entnehmbar ist, findet bei der erfindungsgemäßen Zusammensetzung keine Konversion zu einer anderen Phase der Calciumphosphat-Familie statt.

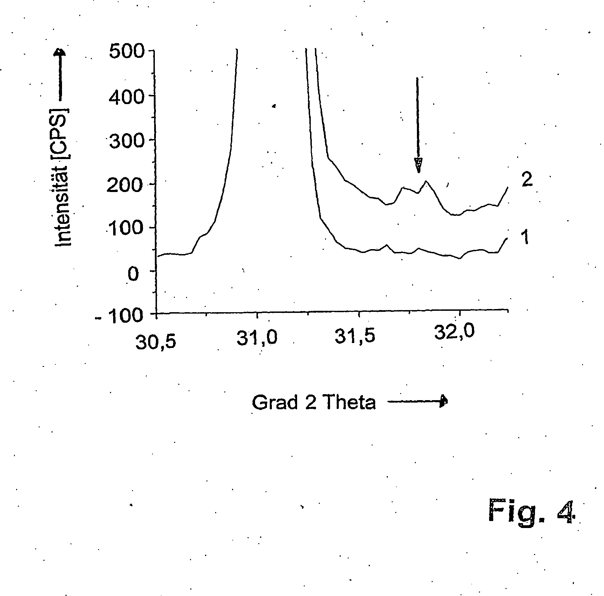

Figur 4 zeigt Detailaufnahmen der Röntgenpulverdiffraktogramme von

(1) der Ausgangscharge einer ß-TCP-Keramik

(2) derselben Keramik nach 12 Wochen Lagerung in Wasser.

Dieser Dokumentation ist entnehmbar, dass in der wässrigen Suspension eine teilweise Konversion / Umkristallisation von ß-TCP zu Hydroxylapatit stattgefunden hat.

Die erfindungsgemäße Zubereitung kann mit trockener Hitze oder mit Gammastrahlen mit einer Dosisleistung von 8 bis 30 kGy (=Kilogray) sterilisiert werden.

Die für die erfindungsgemäße Zubereitung verwendete Hyaluronsäure bzw. das die Hyaluronsäure- Salze können biotechnologisch hergestellt werden, wobei die die Hyaluronsäure ein Molekulargewicht von 1.500.000 bis 4.500.000 Dalton aufweist und durch das Sterilisieren auf 700.000 bis 2.500.000 Dalton sinkt.

## Ausführungsbeispiele

Die Erfindung soll durch nachstehende Beispiele erläutert werden, ohne die Erfindung auf diese zu beschränken.

Für die Herstellung des plastisch verformbaren Implantatmaterials können die Bestandteile A bis K verwendet werden. Dabei sind:

Bestandteil A:

Natriumhyaluronat, biotechnologisch hergestellt mit einem Molekulargewicht von 2,5 MDa

Bestandteil B:

zu 99% phasenreines ß-Tricalciumphosphat, polygon gebrochen, die Bruchkanten durch Abrasion und anschließendes Brennen keramisch abgerundet mit einer Porosität von 20 + 5 % und einer Porengrößenverteilung von 0,1-50 $\mu$m, einer Schüttdichte von 1,1 + 0,1 g/cm$^3$ und einer Korngröße von <63 $\mu$m ($d_5$o= 15 + 5 $\mu$m)

Bestandteil C:

warmes Wasser (30°C) für Injektionszwecke

Bestandteil D:

Methylcellulose niedriger Viskosität

Bestandteil E:

Methylcellulose hoher Viskosität

Bestandteil F:

Hydroxypropylcellulose hoher Viskosität

Bestandteil G:

Hydroxypropylcellulose niedriger Viskosität

Bestandteil H:

Polyethylenglycol 400

Bestandteil I:

Polyethylenglycol 20.000

Bestandteil K:

Dextran

## Ausführungsbeispiel 1

0,3 g Bestandteil A wird mit 20,05 g Bestandteil C ausgefüllt. Nach einer Quellzeit von 4 Stunden wird das Gel steril filtriert und insgesamt 50 g Bestandteil B zugegeben und innig vermischt. Die Mischung wird in einem Behälter bei 121°C durch trockene Hitze sterilisiert.

Es entsteht eine homogene Mischung pastöser Konsistenz die leicht durch eine Kanüle extrudierbar ist, sich einer Defektsituation durch Kriechen anpasst und durch Lufteinwirkung oberflächlich leicht härtet, was durch einen sanften Luftstrom durch eine Druckluftpistole beschleunigt werden kann. Nach der Hitzesterilisation weist das Material ein Molekulargewicht von 1,7 + 0,5 MDa auf.

Auch nach 4-stündigem Zentrifugieren setzt sich kein Feststoff oder Flüssigkeit ab. Eine extrudierte Paste haftet gut an umliegendem Gewebe. In der Simulation des Körpermilieus (Feuchtekammer unter physiologischen Bedingungen) hält ein extrudierter und oberflächlich angetrockneter Körper seine Form.

## Ausführungsbeispiel 2

225 mg Bestandteil D wird mit 38 mg Bestandteil A vermischt und mit 9,7 g Bestandteil C unter Rühren versetzt. Nach einer Quellzeit von 1 Stunde wird das aufgequollene Gel steril filtriert und insgesamt 22 g Bestandteil B langsam unter Rühren zugegeben und innig vermischt. Die Mischung wird in einem geschlossenen Behälter bei 121°C durch trockene Hitze sterilisiert.

Es entsteht eine homogene Mischung pastöser Konsistenz die leicht durch eine Kanüle extrudierbar ist, sich einer Defektsituation durch Kriechen anpasst und durch Lufteinwirkung oberflächlich leicht härtet, was durch einen sanften Luftstrom durch eine Druckluftpistole beschleunigt werden kann. Auch nach 4-stündigem Zentrifugieren setzt sich kein Feststoff oder Flüssigkeit ab. Eine größere Menge setzt auch bei mehrwöchigem Stehenlassen keine wässrige Lösung an der Oberfläche ab. Eine extrudierte Paste haftet gut an umliegendem Gewebe. In der Simulation des Körpermilieus (Feuchtekammer unter physiologischen Bedingungen) hält ein extrudierter und oberflächlich angetrockneter Körper seine Form.

## Ausführungsbeispiel 3

500 mg Bestandteil G wird mit Bestandteil C auf 10,0 g aufgefüllt. Nach einer Quellzeit von 4 Stunden wird das aufgequollene Gel steril filtriert und insgesamt 22 g Bestandteil B langsam unter Rühren zugegeben und innig vermischt. Die Mischung wird in einem geschlossenen Behälter bei 121°C durch trockene Hitze sterilisiert.

Es entsteht eine homogene pastöse Mischung die leicht durch eine Kanüle extrudierbar ist, sich einer Defektsituation durch Kriechen anpasst und durch Lufteinwirkung oberflächlich leicht härtet, was durch einen sanften Luftstrom durch eine Druckluftpistole beschleunigt werden kann. Auch nach 4-stündigem Zentrifugieren setzt sich kein Feststoff oder Flüssigkeit ab. Eine extrudierte Paste haftet gut an umliegenden Gewebe. In der Simulation des Körpermilieus (Feuchtekammer unter physiologischen Bedingungen) hält ein extrudierter und oberflächlich angetrockneter Körper seine Form.

## Ausführungsbeispiel 4

In 1,9 g Bestandteil H wird steril filtriert. Danach wird langsam unter Rühren 5 g Bestandteil B gegeben.

Es entsteht eine gut kriechfähige Paste, welche durch eine Spritze leicht extrudierbar ist. Das Material kriecht leicht in Defektzwischenräume. Auch nach mehrstündigem Zentrifugieren verbleiben die TCP-Partikel in Suspension. Das Material eignet sich sehr gut als Knochenaufbaumaterial, insbesondere für komplizierte Defektsituationen.

## Ausführungsbeispiel 5

2,0 g Bestandteil I wird mit Bestandteil C auf 10,0 g aufgefüllt und nach Homogenisierung steril filtriert. Nach Zugabe von 25 g Bestandteil B wird das Material intensiv vermischt.

Es entsteht eine gut kriechfähige, durch eine Spritze leicht extrudierbare Paste. Das Material ist kriechfähig, füllt daher Defektzwischenräume ideal aus und haftet am umgebenden Gewebe.

Auch nach mehrstündigem Zentrifugieren verbleiben die TCP-Partikel in Suspension. Das Material eignet sich sehr gut als Knochenaufbaumaterial, insbesondere für komplexe Defektsituationen.

### Ausführungsbeispiel 6

3,0 g Bestandteil K wird mit Bestandteil C auf 10,0 g ausgefüllt. Nach dem Lösen des Bestandteil K wird das Material steril filtriert und 26,3 g Bestandteil B zugegeben.

Es entsteht eine durch eine Spritze gut extrudierbare Paste die formbar ist und nicht von der Kanüle tropft. Durch die Formbarkeit lässt sich das Material gut verschiedenen Defektsituationen anpassen und verbleibt als Platzhalter temporär im Defekt.

Auch nach mehrstündigem Zentrifugieren verbleiben die TCP-Partikel in Suspension. Das Material eignet sich sehr gut als Knochenaufbaumaterial, insbesondere für minimalinvasive Anwendungen.

### Ausführungsbeispiel 7

300 mg Bestandteil D wird mit Bestandteil C auf 10 g aufgefüllt. Nach einer Quellzeit von 1 Stunde werden 25 g Bestandteil B mit Korngrößendurchschnittswerten gemäß unten angegener Tabelle versetzt und innig vermischt. Es entstehen pastenförmige Massen, deren Extrudierbarkeit durch verschiedene Kanülendurchmesser getestet wird. Die Extrudierbarkeit ist in der unten angegebenen Tabelle niedergelegt und zeigt die Eigmrung verschiedener Partikeldurchmesser zur Herstellung einer extrudiierbaren Paste.

| Probe | $D_{10}$ [$\mu$m] | $D_{50}$ [$\mu$m] | $D_{20}$ [$\mu$m] | Extrudierbarkeit durch Kanüle mit Ø 0,8 mm | Extrudierbarkeit durch Kanüle mit Ø 2,0 mm |
|-------|------|------|------|-----|-----|
| 1 | 1,60 | 9,65 | 20,28 | ++ | +++ |
| 2 | 2,20 | 15,23 | 35,77 | + | +++ |
| 3 | 10,14 | 21,15 | 44,42 | - | ++ |
| 4 | 14,89 | 35,52 | 63,60 | - | + |
| 5 | 19,35 | 44,48 | 78,15 | - | - |

+++ = sehr gut (gute und homogene Extrudierbarkeit) ++ = gut bis sehr gut (gute Extrudierbarkeit) + = gut bis mäßig (Zufriedenstellende Extrudierbarkeit, leichte Widerstände durch Verkanten einzelner Partikel spürbar) - = mäßig (ausreichende Exctrudierbarkeit, viele Widerstände durch Verkantungen der Partikel) -- = schwer (geringe Extrudierbarkeit, schnelle Verkantung der Partikel) --- = sehr schwer (keine Extrudierbarkeit mehr, Verkantungen erheblich)

Der Vorteil der erfindungsgemäßen Zusammensetzung ist, dass ein phasen- und sedimmentationsstabiles, plastisch verformbares Implantatmaterial mit intrinsischer Porenbildung bereit gestellt wird, das aufwandgering realisiert werden kann und durch Injektion in einen Knochendefekt eingebracht werden kann.

Bei der erfindungsgemäße Zusammensetzung stabilisieren die in Wasser löslichen flüssigen oder pastösen Komponenten die in Wasser schwerlöslichen biologisch aktiven polygon-gebrochenen abgerundeten Partikel im Größenbereich von 0,1-150 $\mu$m mit Poren im Größenbereich von 0,01-50 $\mu$m derart, dass diese nicht durch einen Auflösungs-/ Rekristallisationsprozess in andere Phasen oder Substanzen umgewandelt werden.

Darüber hinaus bildet bei der erfindungsgemäßen Applikation des pastösen Implantatmaterials / der erfindungsgemäßen Zusammensetzung die wasserlösliche organische Bindersubstanz mit der partikulären biologisch aktiven Substanz ein im applizierten Zustand formstabiles, poröses Haufwerk, wobei die leicht lösliche Komponente in der Defektumgebung durch fibröses Gewebe ausgetauscht wird und dadurch die poröse Partikelpackung formstabil verbleibt und langsam integriert, vaskularisiert und resorbiert wird.

Alle in der Beschreibung, den nachfolgenden Ansprüchen und der Zeichnung dargestellten Merkmale können sowohl einzeln als auch in beliebiger Kombination miteinander erfindungswesentlich sein.

Bezugszeichenliste

1 - Hydrogel-Matrix
2 - Partikel
3 - koaguliertes Blut

**Patentansprüche**

1. Pastöse, durch eine Injektionskanüle applizierbare, plastisch verformbare Zubereitung zum Knochenaufbau / -ersatz auf Basis von Keramik-Suspensionen umfassend eine partikuläre, poröse, in Wasser gering lösliche Komponente und mindestens eine in Wasser oder Alkohol gelöste, flüssige, wachsartige oder gelartige Komponente, **dadurch gekennzeichnet, dass** die partikuläre, in Wasser gering lösliche Komponente Calciumphosphat und/oder eine bioaktive Glaskeramik ist; die in Wasser lösliche, flüssige, wachsartige oder gelartige Komponente aus einer wässrigen oder alkoholischen Lösung einer organischen Bindersubstanz, ausgewählt aus der Gruppe der Alginate, Stärken, Polysaccharide, Cellulosen, modifizierten Cellulosen, Hyaluronsäuren und deren Salze, Gelatine, Kollagene, Schellack, Polyacrylsäuren, wässrigen oder alkoholischen Lösungen hiervon, Dextrane, Polyethylenglycole sowie deren Gemischen besteht; die in Wasser gering lösliche Komponente 60- 98 Gew.-% und die in Wasser oder Alkohol gelöste Komponente 1- 30 Gew.-% beträgt, wobei die Partikel der partikulären Komponente einen Durchmesser in einem Größenbereich aufweisen, der im wesentlichen größer als ein phagocytierbarer Partikel und kleiner als der Durchtrittsdurchmesser von Standard-Injektionskanülen ist und der Anteil der gelösten Komponente im Wasser oder Alkohol 0,1- 3 Gew.-% beträgt; **dadurch gekennzeichnet, dass** die Partikel eine Größe im Bereich von 0,1 bis 150 $\mu$m, Poren im Bereich von 0,01-50 $\mu$m und eine polygon-gebrochene Form mit abgerundeter Sinterstruktur aufweisen.

2. Zubereitung gemäß Anspruch 1, **dadurch gekennzeichnet, dass** die Partikel aus Calciumphosphaten und/oder Natrium- und/oder Kalium- und/oder Calcium- und/oder Silicat-haltigen sauren und/oder neutralen und/oder alkalischen Biogläsern oder Gemischen aus den zuvor genannten bestehen.

3. Zubereitung gemäß Anspruch 1 oder 2, **dadurch gekennzeichnet, dass** die Partikel aus Monocalciumphosphat-Monohydrat, wasserfreiem Monocalciumphosphat, Dicalciumphosphat-dihydrat, wasserfreiem Dicalciumphosphat, ß-Tricalciumphosphat, $\alpha$-Tricalciumphosphat, Tetracalciumphosphat, Whitlockit, Octacalciumphosphat, Hydroxylapatit, Oxyapatit, Carbonatapatit vom Typ A, Carbonatapatit vom Typ B, Calcium-defizientem Hydroxylapatit, amorphem Calciumphosphat, amorphen carbonathaltigen Calciumphosphat oder Gemischen aus den zuvor genannten bestehen.

4. Zubereitung gemäß einem der Ansprüche 1 bis 3, **dadurch gekennzeichnet, dass** die Partikel aus phasenreinem ß-Tricalciumphosphat bestehen.

5. Zubereitung gemäß einem der Ansprüche 1 bis 4, **dadurch gekennzeichnet dass** sie mit trockener Hitze oder mit Gammastrahlen mit einer Dosisleistung von 8 bis 30 kGy sterilisierbar ist.

6. Zubereitung gemäß Anspruch 1, **dadurch gekennzeichnet, dass** die Hyaluronsäure bzw. das die Hyaluronsäure-Salze biotechnologisch hergestellt sind.

7. Zubereitung gemäß Anspruch 6, **dadurch gekennzeichnet, dass** die Hyaluronsäure ein Molekulargewicht von 1.500.000 bis 4.500.000 Dalton aufweist und durch das Sterilisieren auf 700.000 bis 2.500.000 Dalton sinkt.

8. Zubereitung gemäß einem der Ansprüche 1 bis 7, **dadurch gekennzeichnet, dass** die Zubereitung ein Gemisch aus 60-98 Masseprozent Calciumphosphat-Partikeln und 1-30 Masseprozent einer wässrigen oder alkoholischen Lösung von Dextran und/oder Carboxymethyldextran und/oder Hyaluronsäure und/oder Dermatansulfat, Carboxymethylcellulose und/oder oxidierter Cellulose und/oder Gelatine und/oder Mischungen aus diesen, oder Polysaccharidderivaten umfasst.

9. Zubereitung gemäß einem der Ansprüche 1 bis 8, **dadurch gekennzeichnet, dass** die mittleren Partikeldurchmesser $d_{10}$, $d_{50}$ und $d_{90}$ der Partikel sich zum Kanülendurchmesser k folgendermaßen verhalten:

$$k \geq 3/20 * d_{10} + 1/2, \ k \geq 2/25 * d_{50} \ \text{und} \ k \geq 2/25 * d_{90} + 4/5.$$

10. Zubereitung gemäß einem der Ansprüche 1 bis 9, **dadurch gekennzeichnet, dass** die Zubereitung aus einer Mischung von 60-80 Gew.-%, vorzugsweise 67-75 Gew.-% ß-Tricalciumphosphat, 17-37 Gew.-%, vorzugsweise 25-35 Gew.-% Wasser und 0,3-3 Gew.-%, vorzugsweise 0,5-1,5 Gew.-% Methylcellulose niedriger Viskosität besteht.

11. Zubereitung gemäß einem der Ansprüche 1 bis 9, **dadurch gekennzeichnet, dass** die Zubereitung aus einer Mischung von 60-80 Gew.-%, vorzugsweise 68-76 Gew.-% ß- Tricalciumphosphat, 15-35 Gew.~%, vorzugsweise 25-31 Gew.-% Wasser und 0,1-3 Gew.-%, vorzugsweise 0,2-0,9 Gew.-% Methylcellulose niedriger Viskosität und 0,01-2 Gew.-%, vorzugsweise 0,1-0,6 Gew.-% Natriumhyaluronat oder Hyaluronsäure besteht.

12. Zubereitung gemäß einem der Ansprüche 1 bis 9, **dadurch gekennzeichnet, dass** die Zubereitung aus einem Gemisch aus 80-98 Masseprozent Calciumphosphat-Partikeln, 1-20 Masseprozent wasserfreiem Polyethylenglykol 400 und 1-20 Masseprozent wasserfreiem Polyethylenglykol 600 besteht.

13. Zubereitung gemäß einem der Ansprüche 1 bis 12, **dadurch gekennzeichnet, dass** die Zubereitung eine Dichte zwischen 1,3 $g/cm^3$ und 2,1 $g/cm^3$, vorzugsweise zwischen 1,6 $g/cm^3$ und 1,9 $g/cm^3$ besitzt.

14. Zubereitung gemäß einem der Ansprüche 1 bis 13, **dadurch gekennzeichnet, dass** sie wachstumsinduzierende Proteine enthält.

15. Zubereitung gemäß einem der Ansprüche 1 bis 14, **dadurch gekennzeichnet, dass** sie Wachstumsfaktoren aus der Familie der Growth Factors (GF) oder der Bone Morphogenese Proteins enthält.

16. Zubereitung gemäß Anspruch 15, **dadurch gekennzeichnet, dass** die Wachstumsfaktoren BMP-1 bis BMP-12, besonders vorteilhaft BMP-2 und BMP-7, und/oder FGF, TGF-ß, PDGF, VEGF, IGF, HGF, PTH und / oder Gemische aus diesen sind.

17. Zubereitung gemäß einem der Ansprüche 1 bis 16, **dadurch gekennzeichnet, dass** sie Platelet Rieh Plasma (PRP) enthält.

18. Verfahren zur Herstellung einer Zubereitung gemäß einem der Ansprüche 1 bis 17, **dadurch gekennzeichnet, dass** die Partikel mit einem Durchmesser in einem Größenbereich, der überwiegend größer als ein phagocytierbarer Partikel und der auf den jeweiligen Durchtrittsdurchmesser von gängigen Injektionskanülen angepasst ist, verwendet und mit einer Bindersubstanzen unter gleichzeitigem Zusatz stabilisierender, die Sedimentation unterdrückender Stoffe, innig vermischt werden.

19. Verfahren zur Herstellung einer Zubereitung gemäß Anspruch 18, **dadurch gekennzeichnet, dass** 80-98 Masseprozent Calciumphosphat- Partikeln, 1-20 Masseprozent wasserfreiem Polyethylenglykol 400 und 1-20 Masseprozent wasserfreiem Polyethylenglykol 600 vermischt werden.

20. Verfahren zur Herstellung einer Zubereitung gemäß Anspruch 19, **dadurch gekennzeichnet, dass** Polyethylenglykol 400 und Polyethylenglykol 600 zusätzlich Oxidationsstabilisatoren enthalten.

21. Verfahren nach Anspruch 18, **dadurch gekennzeichnet, dass** eine oder mehrere wasserlösliche oder in alkoholischen Lösungsmitteln lösliche Hydrogel-bildende Komponenten in ein Lösungsmittel gegeben wird, nach einer Quellzeit steril filtriert wird, danach mit einer biologisch aktiven Substanz unter Rühren vermischt wird, nach mehrstündigem Rühren in dicht verschließende Applikationssysteme abgefüllt wird und 10-60 min bei 100-130°C unter Hitze sterilisiert wird.

**Claims**

1. A paste-like plastically deformable preparation for bone construction/replacement based on ceramic suspensions that can be applied using an injection cannula, comprising a particulate, porous component that is not easily soluble in water and at least one liquid, wax-like, or gel component that is dissolved in water or alcohol, **characterized in that** the particulate component that is not easily soluble in water is calcium phosphate and/or a bioactive glass ceramic; the liquid, wax-like, or gel component that is soluble in water consists of an aqueous or alcoholic solution of an organic binder substance selected from the group of alginates, starches, polysaccharides, celluloses, modified celluloses, hyaluronic acids and their salts, gelatins, collagens, shellac, polyacrylic acids, aqueous or alcoholic solutions thereof, dextrans, polyethylene glycols, or mixtures thereof; the component that is not easily soluble in water is 60 - 98% by weight and the component dissolved in water or alcohol is 1 - 30% by weight, wherein the particles of the particulate component having a diameter in a size range that is essentially greater than a phagocytable particle and smaller than the passage diameter of standard injection cannulas and the portion of the dissolved component in the water or alcohol being 0.1 - 3% by weight; **characterized in that** the particles have a size in the range of 0.1 to 150 $\mu$m, pores in the range of 0.01 - 50 $\mu$m, and a polygonally broken shape with a rounded sintered structure.

2. The preparation in accordance with claim 1, **characterized in that** the particles consist of calcium phosphates and/or sodium-containing and/or potassium-containing and/or calcium-containing and/or silicate-containing acid and/or neutral and/or alkaline bioglasses or mixtures of the aforesaid.

3. The preparation in accordance with claim 1 or 2, **characterized in that** the particles consist of monocalcium phosphate-monohydrate, anhydrous monocalcium phosphate, dicalcium phosphate-dihydrate, anhydrous dicalcium phosphate, $\beta$-tricalcium phosphate, a -tricalcium phosphate, tetracalcium phosphate, whitlockite, octacalcium phosphate, hydroxyapatite, oxyapatite, type A carbonate apatite, type B carbonate apatite, calcium-deficient hydroxyapatite, amorphous calcium phosphate, amorphous carbonate-containing calcium phosphate, or mixtures of the aforesaid.

4. The preparation in accordance with any one of claims 1 to 3, **characterized in that** the particles consist of phase-pure $\beta$-tricalcium phosphate.

5. The Preparation in accordance with any one of claims 1 to 4, **characterized in that** it can be sterilized with dry heat or with gamma rays at a dosage of 8 to 30 kGy.

6. The preparation in accordance with claim 1, **characterized in that** the hyaluronic acid or **in that** the hyaluronic acid salts are bioengineered.

7. The preparation in accordance with claim 6, **characterized in that** the hyaluronic acid has a molecular weight of 1,500,000 to 4,500,000 Daltons and is reduced to 700,000 to 2,500,000 Daltons by sterilization.

8. The preparation in accordance with any one of claims 1 to 7, **characterized in that** the preparation comprises a mixture of 60 - 98% by mass calcium phosphate particles and 1-30% by mass of an aqueous or alcoholic solution of dextran and/or carboxymethyl dextran and/or hyaluronic acid and/or dermatan sulfate, carboxymethyl cellulose and/or oxidized cellulose and/or gelatins and/or mixtures thereof, or polysaccharide derivatives.

9. The preparation in accordance with any one of claims 1 to 8, **characterized in that** the mean particle diameters $d_{10}$, $d_{50}$, and $d_{90}$ of the particles behave as follows for cannula diameter k:

$$k \geq 3/20 * d_{10} + l/2, \quad k \geq 2/25 * d_{50} \text{ und } k \geq 2/25 * d_{90} + 4/5.$$

10. The preparation in accordance with any one of claims 1 to 9, **characterized in that** the preparation consists of a mixture of 60 - 80% by weight, preferably 67 - 75% by weight $\beta$-tricalcium phosphate, 17 - 37% by weight, preferably 25 - 35% by weight water, and 0.3 - 3% by weight, preferably 0.5 - 1.5% by weight low-viscosity methylcellulose.

11. The preparation in accordance with any one of claims 1 to 9, **characterized in that** the preparation consists of a mixture of 60 - 80% by weight, preferably 68 - 76% by weight $\beta$-tricalcium phosphate, 15 - 35% by weight, preferably 25 - 31% by weight water, and 0.1 - 3% by weight, preferably 0.2 - 0.9% by weight low-viscosity methylcellulose and 0.01 - 2% by weight, preferably 0.1 - 0.6% by weight sodium hyaluronate or hyaluronic acid.

**12.** The preparation in accordance with any one of claims 1 to 9, **characterized in that** the preparation consists of a mixture of 80 - 98% by mass calcium phosphate particles, 1 - 20% by mass anhydrous polyethylene glycol 400, and 1 - 20% by mass anhydrous polyethylene glycol 600.

**13.** The preparation in accordance with any one of claims 1 to 12, **characterized in that** the preparation has a density between 1.3 g/cm$^3$ and 2.1 g/cm$^3$, preferably between 1.6 g/cm$^3$ and 1.9 g/cm$^3$.

**14.** The preparation in accordance with any one of claims 1 to 13, **characterized in that** it contains growth-inducing proteins.

**15.** The preparation in accordance with any one of claims 1 to 14, **characterized in that** it contains growth factors from the family of growth factors (GF) or from the bone morphogenetic proteins.

**16.** The preparation in accordance with claim 15, **characterized in that** the growth factors are BMP-1 through BMP-12, particularly advantageously BMP-2 and BMP-7, and/or FGF, TGF-$\beta$, PDGF, VEGF, IGF, HGF, PTH, and/or mixtures thereof.

**17.** The preparation in accordance with any one of claims 1 to 16, **characterized in that** it contains platelet rich plasma (PRP).

**18.** A method for producing a preparation in accordance with any one of claims 1 to 17, **characterized in that** particles are used that have a diameter in a range that is predominantly greater than a phagocytable particle and that is matched to the passage diameter of common injection cannulas and these particles are mixed intimately with a binder substance(s), stabilizing substances that suppress sedimentation being added simultaneously.

**19.** The method for producing a preparation in accordance with claim 18, **characterized in that** 80 - 98% by mass calcium phosphate particles, 1 - 20% by mass anhydrous polyethylene glycol 400, and 1 - 20% by mass anhydrous polyethylene glycol 600 are mixed.

**20.** The method for producing a preparation in accordance with claim 19, **characterized in that** polyethylene glycol 400 and polyethylene glycol 600 additionally contain oxidation stabilizers.

**21.** The method according to claim 18, **characterized in that** one or more hydrogel-forming components that are water-soluble or that are soluble in alcoholic solvents are added to a solvent, sterile-filtration is carried out after a swelling time, then are mixed with a biologically active substance while stirring, after multiple hours of stirring are filled in tightly sealing application systems, and sterilization is carried out with heat for 10 - 60 min at 100 - 130°C.

**Revendications**

**1.** Préparation pâteuse, applicable par une canule d'injection, déformable plastiquement, destinée à la construction/substitution osseuse, à base de suspensions de céramiques comprenant une composante particulaire, poreuse, peu soluble dans l'eau, et au moins une composante solubilisée dans l'eau ou l'alcool, liquide, de type cire ou gel, **caractérisée en ce que** la composante particulaire, peu soluble dans l'eau, est du phosphate de calcium et/ou une vitrocéramique bioactive ; la composante soluble dans l'eau, de type cire ou gel, consiste en une solution aqueuse ou alcoolique d'une substance liante organique, choisie dans le groupe des alginates, des amidons, des polysaccharides, des celluloses, des celluloses modifiées, des acides hyaluroniques et de leurs sels, de la gélatine, des collagènes, de la gomme-laque, des acides polyacryliques, de leurs solutions aqueuses ou alcooliques, des dextranes, des polyéthylène glycols ainsi que de leurs mélanges ; la composante peu soluble dans l'eau s'élève à 60 - 98 % en poids et la composante solubilisée dans l'eau ou l'alcool s'élève à 1 - 30 % en poids, les particules de la composante particulaire présentant un diamètre dans un intervalle de taille qui est essentiellement supérieur à celui d'une particule pouvant être phagocytée et inférieur au diamètre moyen des canules d'injection standard et la proportion des composantes solubilisées dans l'eau ou dans l'alcool s'élève à 0,1 - 3 % en poids ; **caractérisée en ce que** les particules possèdent une taille dans l'intervalle de 0,1 à 150 $\mu$m, des pores dans l'intervalle de 0,01 - 50 $\mu$m et une forme en polygone tronqué avec une structure frittée arrondie.

**2.** Préparation selon la revendication 1, **caractérisée en ce que** les particules sont constituées de phosphates de calcium et/ou d'acides contenant du sodium, et/ou du potassium et/ou du calcium et/ou un silicate et/ou de verres biologiques neutres et/ou alcalins ou de mélanges des produits nommés précédemment.

3. Préparation selon a revendication 1 ou 2, **caractérisée en ce que** les particules sont constituées de phosphate monocalcique monohydraté, de phosphate monocalcique exempt d'eau, de phosphate dicalcique dihydraté, de phosphate dicalcique exempt d'eau, de phosphate tricalcique β, de phosphate tricalcique α, de phosphate tétracalcique, de whitlockite, de phosphate octacalcique, d'hydroxylapatite, d'oxyapatite, de carbonapatite de type A, de carbonapatite de type B, d'hydroxylapatite déficiente en calcium, de phosphate de calcium amorphe, de phosphate de calcium contenant du carbone amorphe ou de mélanges des produits cités précédemment.

4. Préparation selon l'une des revendications 1 à 3, **caractérisée en ce que** les particules sont constituées de phosphate tricalcique β de phase pure.

5. Préparation selon l'une des revendications 1 à 4, **caractérisée en ce qu'**elle peut être stérilisée par la chaleur sèche ou par des rayons gamma avec un débit de dose entre 8 et 30 kGy.

6. Préparation selon la revendication 1, **caractérisée en ce que** de l'acide hyaluronique, respectivement, des sels d'acide hyaluronique, sont fabriqués par biotechnologie.

7. Préparation selon la revendication 6, **caractérisée en ce que** l'acide hyaluronique présente une masse moléculaire entre 1 500 000 et 4 500 000 Dalton et qu'elle chute jusqu'à 700 000 et 2 500 000 Dalton.

8. Préparation selon l'une des revendications 1 à 7, **caractérisée en ce que** la préparation comprend un mélange de 60 - 98 pour cent en masse de particules de phosphate de calcium et 1 - 30 pour cent en masse d'une solution aqueuse ou alcoolique de dextrane et/ou de carboxyméthyldextrane et/ou d'acide hyaluronique et/ou de dermatan sulfate, de carboxyméthylcellulose et/ou de cellulose oxydée et/ou des gélatines et/ou de mélanges de ceux-ci ou de dérivés de polysaccharides.

9. Préparation selon l'une des revendications 1 à 8, **caractérisée en ce que** les diamètres moyens de particules $d_{10}$, $d_{50}$ et $d_{90}$ des particules se présentent de la manière suivante par rapport au diamètre des canules k :

$$k \geq 3/20*d_{10} + 1/2, \ k \geq 2/25*d_{50} \ et \ k \geq 2/25*d_{90} + 4/5.$$

10. Préparation selon l'une des revendications 1 à 9, **caractérisée en ce que** la préparation est constituée d'un mélange de 60 - 80 % en poids, de préférence, de 67 - 75 % en poids, de phosphate tricalcique β, de 17 - 37 % en poids, de préférence, de 25 - 35 % en poids, d'eau et de 0,3 - 3 % en poids, de préférence, de 0,5 - 1,5 % en poids, de méthylcellulose de faible viscosité.

11. Préparation selon l'une des revendications 1 à 9, **caractérisée en ce que** la préparation est constituée d'un mélange de 60 - 80 % en poids, de préférence, de 68 - 76 % en poids, de phosphate tricalcique β, de 15 - 35 % en poids, de préférence, de 25 - 31 % en poids, d'eau et de 0,1 - 3 % en poids, de préférence, de 0,2 - 0,9 %en poids, de méthylcellulose de faible viscosité et de 0,01 - 2 % en poids, de préférence, de 0,1 - 0,6 % en poids, d'hyaluronate de sodium ou d'acide hyaluronique.

12. Préparation selon l'une des revendications 1 à 9, **caractérisée en ce que** la préparation est constituée d'un mélange de 80 - 98 % en masse de particules de phosphate calcique, de 1 - 20 % en masse de polyéthylène glycol 400 exempt d'eau et de 1 - 20 % en masse de polyéthylène glycol 600 exempt d'eau.

13. Préparation selon l'une des revendications 1 à 12, **caractérisée en ce que** la préparation possède une densité entre 1,3 g/cm$^3$ et 2,1 g/cm$^3$, de préférence entre 1,6 g/cm$^3$ et 1,9 g/cm$^3$.

14. Préparation selon l'une des revendications 1 à 13, **caractérisée en ce qu'**elle contient une protéine induisant la croissance.

15. Préparation selon l'une des revendications 1 à 14, **caractérisée en ce qu'**elle contient des facteurs de croissance de la famille des facteurs de croissance (GF) ou des protéines de morphogénèse osseuse.

**16.** Préparation selon la revendication 15, **caractérisée en ce que** les facteurs de croissance sont les facteurs BMP-1 à BMP-12, BMP-2 et BMP-7 étant particulièrement avantageux, et/ou FGF, TGF-β, PDGF, VEGF, IGF, HGF, PTH et/ou des mélanges de ceux-ci.

**17.** Préparation selon l'une des revendications 1 à 16, **caractérisée en ce qu'**elle contient du plasma riche en plaquettes (PRP).

**18.** Procédé de fabrication d'une préparation selon l'une des revendications 1 à 17, **caractérisée en ce que** la particule est employée avec un diamètre dans un domaine de taille qui est dans l'ensemble supérieur à celui d'une particule pouvant être phagocytée et qui s'adapte au diamètre de passage courant des canules d'injection usuelles, et est mélangée intimement avec une substance liante moyennant l'ajout simultané de substances stabilisantes, réprimant la sédimentation.

**19.** Procédé de fabrication d'une préparation selon la revendication 18, **caractérisé en ce que** 80 - 98 pour cent en masse de particules de phosphate de calcium, 1 - 20 pour cent en masse de polyéthylène glycol 400 exempt d'eau et 1 - 20 pour cent en masse de polyéthylène glycol 600 exempt d'eau sont mélangés.

**20.** Procédé de fabrication d'une préparation selon la revendication 19, **caractérisé en ce que** le polyéthylène glycol 400 et le polyéthylène glycol 600 contiennent des stabilisants d'oxydation en excédent.

**21.** Procédé selon la revendication 18, **caractérisé en ce qu'**une ou plusieurs composante(s) formant un hydrogel soluble dans l'eau ou dans des solvants alcooliques est(sont) ajoutée(s) dans un solvant, après un moment de gélification, elle(s) est(son) filtrée(s) de manière stérile, puis est(sont) mélangée(s) sous agitation avec une substance biologiquement active, après une agitation de plusieurs heures, elle(s) est(sont) vidée(s) dans des systèmes d'application hermétiquement fermés et stérilisée(s) par la chaleur à 100 - 130 °C pendant 10 - 60 minutes.

Fig. 1

Fig. 2

Fig. 3

EP 2 059 269 B1

Fig. 4

20

Fig. 5

EP 2 059 269 B1

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente

- WO 03063686 A
- EP 1477176 A1
- US 5258028 A
- US 471076 A
- US 4780450 A
- EP 0416398 A1
- US 2002169506 A
- WO 03082365 A
- US 2003055512 A
- WO 2004011053 A
- FR 2852249
- WO 03035124 A
- WO 03028779 A
- WO 02058755 A
- WO 0141821 A
- EP 1475109 A1
- WO 0141824 A
- WO 0007639 A
- WO 0045867 A
- WO 9521634 A
- US 5352715 A

### In der Beschreibung aufgeführte Nicht-Patentliteratur

- **R. MURUGAN ; S. RAMAKRISHNA.** Bioresorbable composite bone paste using Polysaccharide based nano hydroxyapatite. *Biomaterials,* 2004, vol. 25, 3829-3835
- **F. PETERS ; D. REIF.** Functional Materials for Bone Regeneration from Beta-Tricalcium Phosphate. *Mat.-wiss. u. Werkstofftech,* 2004, vol. 35 (4), 203-207
- **S. SHIMIZU.** *Biomed. Res.,* 1988, vol. 9 (2), 95
- **J. VAN DER MEULEN ; H. K. KOERTEN.** *J. Biomed. Mater. Res.,* 1994, vol. 28, 1455